Europäisches Patentamt

(19) European Patent Office

Office européen des brevets

(11) Publication number : **0 332 382 B1**

(12) **EUROPEAN PATENT SPECIFICATION**

(45) Date of publication of patent specification :
**09.12.92 Bulletin 92/50**

(51) Int. Cl.$^5$ : **A61K 7/06,** A61K 7/48

(21) Application number : **89302235.0**

(22) Date of filing : **06.03.89**

(54) **Preparation for use on the hair and body.**

(30) Priority : **07.03.88 GB 8805369**

(43) Date of publication of application :
**13.09.89 Bulletin 89/37**

(45) Publication of the grant of the patent :
**09.12.92 Bulletin 92/50**

(84) Designated Contracting States :
**AT BE CH DE ES FR GR IT LI NL SE**

(56) References cited :
**FR-A- 855 537**
**FR-A- 936 668**
**GB-A- 240 578**

(56) References cited :
**GB-A- 281 425**
**GB-A- 413 370**
**GB-A- 439 011**
**GB-A- 1 237 656**
**GB-A- 1 537 956**

(73) Proprietor : **Clachar, Carrol Joseph**
**76B, Endlesham Road**
**Balham London SW12 (GB)**

(72) Inventor : **Clachar, Carrol Joseph**
**76B, Endlesham Road**
**Balham London SW12 (GB)**

(74) Representative : **Pedder, James Cuthbert**
**J.C. Pedder & Co. 38 Norbury Cross**
**Norbury London SW16 4JQ (GB)**

EP 0 332 382 B1

## Description

This invention relates to a preparation for use on the hair and body.

Many preparations have appeared on the market restoring the natural oils to the hair and to the body which for many reasons today are no longer present in the usual quantities. This may be as a result of health or advancing age. In the case of hair, this condition may be due to mistreatment of the hair itself. Furthermore, in addition to this, baldness, particularly in men, has always been a problem and various treatments and preparations have been proposed to treat or mitigate this problem. Unfortunately, many of these preparations have not been significantly successful and a number have had no effect whatsoever.

Cosmetic products for hair or skin are disclosed in FR-A-855 537, GB-A-1 537 956 and FR-A-936 668.

The present invention seeks to provide a preparation for use on the hair or body which will not only restore natural oils to the hair or body but which will, when applied to the hair or scalp, stimulate hair growth.

According to the invention, a preparation for use on the hair or body comprises a lotion including by weight 41 - 51 parts glycerine, 38 - 48 parts rosewater, 3 - 5 parts liquid paraffin and 2 - 4 parts olive oil.

Preferably the lotion comprises, by weight, 43 - 49 parts glycerine, 40 - 46 parts rosewater, 4 parts liquid paraffin and 3 parts olive oil.

To this basic lotion may be added further compounds of the order of 2 - 4 parts of one or more substances to increase the body of the lotion, up to 1 part of a fragrance and up to 1 part of a colouring.

The invention will now be described in greater detail, by way of example, with reference to a particularly effective and safe form of preparation in accordance with the invention.

Then particular form of lotion now to be described has all the advantages stated above when applied to the hair, scalp and other bare skin areas of the body. It is, however not recommended for use on broken areas of the skin or open wounds though it is believed that no ill effects should be found even in these circumstances. Additionally, the components are such that the lotion is entirely non toxic and, if taken internally, will have no ill effects.

This preferred form of lotion comprises the following percentages by weight:

| | |
|---|---|
| Glycerine | 46 |
| Rosewater | 43 |
| Liquid paraffin | 4 |
| Olive oil | 3 |
| Almond oil | 2 |
| Petroleum jelly | 1 1/3 |
| Fragrance of Florida water | 1/3 |
| Edible colouring | 1/3 |

In preparation of the lotion, the various ingredients are mixed together in the proportions indicated at room temperature. Because some of the components are imiscible liquids, there is some separation, but this can be dealt with by shaking the lotion before use. Alternatively, mixing can take place in an emulsifier to produce an emulsified product.

While the various components serve to carry out effects specific to the components concerned, the particular proportions (or range of proportions) of the components of the present invention have particularly effective properties which are surprisingly greater than could be expected from the properties and proportions of the individual components. In particular, the lotion has been found to be effective in reducing or curing alopecia prematura and to be helpful in cases of eczema and similar skin complaints.

It is to be understood that, the above described lotion is the preferred form of the lotion.

Basically, the invention requires, to be effective, only the glycerine, rosewater, liquid paraffin and olive oil components in the ranges stated above. Of these, the glycerine serves as a softener, the rosewater for grease removal and the liquid paraffin and olive oil are moisturisers. The other components can be added as desired to increase the effectiveness, to provide a suitable fragrance and to provide suitable colouring of the lotion.

It will be appreciated that, taking the specific example described above, the lotion will remain effective within a range of ± 10% though to a lesser extent as the range approaches its limits.

It will also be appreciated that certain of the components can be replaced by other components. For example, almond oil could be replaced by coconut oil or castor oil and the fragrance of Florida water could be replaced by Oil of Spikes or Kananga Water. Other edible colourings could be used to give different coloured lotions.

## Claims

1. A preparation for use on the hair and body comprising a lotion including by weight 41 - 51 parts glycerine, 38 - 48 parts rosewater, 3 - 5 parts liquid paraffin and 2 - 4 parts olive oil.

2. A preparation as claimed in claim 1, wherein the lotion comprises, by weight, 43 - 49 parts glycerine, 40 - 46 parts rosewater, 4 parts liquid paraffin and 3 parts olive oil.

3. A preparation as claimed in claim 1 or 2, wherein to the lotion is added 2 - 4 parts of one or more substances to increase the body of the lotion.

4. A preparation as claimed in claim 1, 2 or 3, wherein to the lotion is added up to 1 part of a fragrance.

5. A preparation as claimed in any one of claims 1 to 4, wherein to the lotion is added up to 1 part of a colouring.

6. A preparation as claimed in any any one of claims 1 to 5, wherein the lotion has the following composition in percentage weights:

| | |
|---|---|
| Glycerine | 46 |
| Rosewater | 43 |
| Liquid paraffin | 4 |
| Olive oil | 3 |
| Almond oil | 2 |
| Petroleum jelly | 1 1/3 |
| Fragrance of Florida water | 1/3 |
| Edible colouring | 1/3 |

7. A preparation as claimed in any one of claims 1 to 6, wherein the lotion is in the form of an emulsion.

## Patentansprüche

1. Präparat zur Verwendung für Haare und Körper, das eine Lotion umfaßt, die auf das Gewicht bezogen 41-51 Teile Glyzerin, 38-48 Teile Rosenwasser, 3-5 Teile flüssiges Paraffin und 2-4 Teile Olivenöl umfaßt.

2. Präparat nach Anspruch 1, worin die Lotion auf das Gewicht bezogen 43-49 Teile Glyzerin, 40-48 Teile Rosenwasser, 4 Teile flüssiges Paraffin und 3 Teile Olivenöl umfaßt.

3. Präparat nach Anspruch 1 oder 2, worin der Lotion 2-4 Teile einer oder mehrerer Substanzen zugesetzt werden, um die Substanz der Lotion zu verbessern.

4. Präparat nach Anspruch 1, 2 oder 3, worin der Lotion bis zu 1 Teil eines Duftstoffes zugesetzt wird.

5. Präparat nach einem der Ansprüche 1 bis 4, worin der Lotion bis zu 1 Teil eines Farbstoffs zugesetzt wird.

6. Präparat nach einem der Ansprüche 1 bis 5, worin die Lotion die folgende Zusammensetzung in Gew.-% aufweist:

| | |
|---|---|
| Glyzerin | 46 |
| Rosenwasser | 43 |
| flüssiges Paraffin | 4 |
| Olivenöl | 3 |
| Mandelöl | 2 |
| Rohvaseline | 1 1/3 |
| Duftstoff "Floridawasser" | 1/3 |
| Lebensmittelfarbe | 1/3 |

7. Präparat nach einem der Ansprüche 1 bis 6, worin die Lotion in Form einer Emulsion vorliegt.

**Revendications**

1. Préparation destinée à l'utilisation sur les cheveux et sur le corps, comprenant une lotion incorporant 41 - 51 parties en poids de glycérine, 38 - 48 parties en poids d'eau de rose, 3 - 5 parties en poids de paraffine liquide et 2 - 4 parties en poids d'huile d'olive.

2. Préparation selon la revendication 1, dans laquelle la lotion comprend 43 - 49 parties en poids de glycérine, 40 - 46 parties en poids d'eau de rose, 4 parties en poids de paraffine liquide et 3 parties en poids d'huile d'olive.

3. Préparation selon la revendication 1 ou 2, dans laquelle on ajoute à la lotion 2 - 4 parties d'une ou plusieurs des substances pour augmenter le corps de la lotion.

4. Préparation selon la revendication 1, 2 ou 3, dans laquelle on ajoute à la lotion jusqu'à 1 partie d'un parfum.

5. Préparation selon l'une quelconque des revendications 1 à 4, dans laquelle on ajoute à la lotion jusqu'à 1 partie d'un colorant.

6. Préparation selon l'une quelconque des revendications 1 à 5, dans laquelle la lotion comporte la composition suivante en pourcentage en poids :

| Glycérine | 46 |
| Eau de rose | 43 |
| Paraffine liquide | 4 |
| Huile d'olive | 3 |
| Huile d'amandes | 2 |
| Pétrolatum | 1 1/3 |
| Parfum d'eau de Floride | 1/3 |
| Colorant comestible | 1/3 |

7. Préparation selon l'une quelconque des revendications 1 à 6, dans laquelle la lotion se présente sous forme d'une émulsion.